# EUROPEAN PATENT APPLICATION

(11) **EP 1 632 253 A1**
(43) Date of publication of application: **08.03.2006**
(21) Application number: 05388064.7
(22) Date of filing: 03.08.2005
(51) Int. Cl.: A61L 15/46, D06M 23/00

(54) **Material for odour control**

(30) Priority: 04.08.2004 US 598867 P; 25.07.2005 US 702504 P
(71) Applicant: BKI Holding Corporation, Wilmington, Delaware 19801 (US)
(72) Inventor: Dutkiewicz, Jacek K., Cordova TN 38016 (US)
(74) Representative: Nilausen, Kim

(57) **Abstract**

The present invention is directed to materials containing an enzyme inhibitor, including treated fibers, nonwovens, functional particles and processes for making these materials. These materials are useful in reducing or delaying onset of odor generation from various waste materials including human and animal elimination products.

## Description

This application claims the benefit of U.S. Provisional Application No. 60/598,867, filed August 4, 2004 and U.S. Provisional Application No. 60/---,---, filed July 25, 2005. Each of these applications are hereby incorporated by reference.

### FIELD OF THE INVENTION

This invention relates to materials which can reduce or delay the evolution of offensive odors from personal care articles, which while in use are exposed to and contain various body exudates and elimination products such as feces and urine.

### BACKGROUND OF THE INVENTION

A continuing problem in the state of the art related to personal care articles such as disposable diapers, panty liners, feminine napkins and pads, and incontinent devices, is that if not disposed of relatively soon after being soiled, offensive odors become an issue. A major aspect of this issue is the formation of ammonia by the decomposition of urea, which is accelerated by urease.

Previous attempts at addressing this issue have frequently involved the use of ingredients to absorb or chemically bind the offensive gaseous materials after they have been produced. U.S. Patent No. 6,706,941 discloses the use of glycooligosaccharides. U.S. Patent Nos. 6,689,378, 6,433,243, and 6,229,062 disclose cyclodextrins, while U.S. Patent Nos. 5,161,686, 5,306,487 and 6,096,299 use zeolite; U.S. Patent No. 6,175,055 uses bentonite; U.S. Patent No. 6,652,845 uses acids; U.S. Patent No. 5,407,442 uses activated carbon with zeolites; and U.S. Patent Nos. 4,676,196, 5,306,487, and 6,369,290 use sodium carbonate.

Inhibition is used in fertilizer applications where premature decomposition of urea is the issue. U.S. Patent Nos. 4,517,005, 4,517,007 and 4,530,714, hereby incorporated herein by reference in their entirety, relate to various urease inhibitors and their use of urea based fertilizer compositions. U.S. Patent No. 6,287,550, hereby incorporated herein by reference in its entirety, relates to the use of urease inhibition in combination with odor absorption in animal litter. U.S. Patent Application number 2003/0120228, hereby incorporated herein by reference in its entirety, discloses the use of yucca extract as a urease inhibitor. U.S. Patent Application number 2004/0116882, hereby incorporated herein by reference in its entirety, discloses particular EDTA salts which can be used in a coating in an absorbent article to control odor without discoloration.

U.S. Patent 6,703,536, hereby incorporated herein by reference in its entirety, discloses an absorbent article, at least a portion of which comprises a skin care composition that comprises an enzyme inhibitor. The skin care composition, including the enzyme inhibitor, is at least partially transferred from the absorbent article to the wearer's skin as a result of normal contact, wearer motion and/or body heat. Since the skin care composition with the enzyme inhibitor is transferred to the skin, the inhibitor is available at the skin/urine and skin/feces interface to inhibit enzymatic activity on the skin and reduce or prevent the occurrence of inflammation. Repeated application of similarly treated articles to the wearer's skin provides an available source with which the enzyme inhibitor transfers onto the skin continuously over time and accumulates to provide a proactive defense against harmful enzymes for the treatment and/or prevention of diaper dermatitis.

It would be desirable to have available, for use in the field of personal care products which are exposed to bodily wastes, fibrous materials which, when exposed to these wastes, inhibit the decomposition of the waste materials which give rise to offensive odors.

### SUMMARY OF THE INVENTION

The present invention advantageously provides a treated fiber capable of controlling offensive odors. Specifically, the fiber can be used in fibrous materials including absorbent articles or may be used in liquid form.

The present invention provides a treated fiber comprising fiber and, based on the weight of the treated fiber, from about 0.0001 weight percent to about 10 weight percent of an available enzyme inhibitor. The inhibitory effectiveness of the treated fiber is about 75 percent or greater Within the scope of one aspect of this invention is a process for the production of a treated fiber comprising contacting fibers with from about 0.0001 weight percent to about 10 weight percent of an available enzyme inhibitor based on the weight of the treated fiber. The fiber may be individualized or may be in the form of a comminution sheet. Additionally, the fibers may be in the form of a nonwoven material when contacted with the enzyme inhibitor.

In one embodiment, the enzyme inhibitor inhibits proteolytic enzymes, including but not limited to trypsin, chymotrypsin, aminopeptidase, elastase, lipases, bile salts, amylases, ureases, or a combination thereof.

In another embodiment, the enzyme inhibitor is a chelating agent. In another embodiment, the enzyme inhibitor is a protease inhibitor, a lipase inhibitor, a bile salt inhibitor, an amylase inhibitor, a glucosidase inhibitor, or a combination thereof. In other embodiments, the enzyme inhibitor is a urease inhibitor, a metal salt, or a transition metal ion salt. The salts may contain zinc, aluminum, or zinc ions. The metal salts have a metal ion content of from about 0.0005 weight percent to about 5 weight percent based on the weight of the treated fiber, preferably from about 0.0005 weight percent to about 3 weight percent, preferably from about 0.0005 weight percent to about 2 weight percent, more preferably from about 0.001 weight percent to about 2 weight percent, and more preferably from about 0.01 weight percent to about 2 weight percent based on the weight of the treated fiber. Metal salts of the invention are present in an amount from about 10 ppm to about 10,000 ppm.

In a specific embodiment, the enzyme inhibitor is zinc chloride, zinc sulfate or a mixture thereof. In other embodiments, the enzyme inhibitor is an ammonium thiosulfate or a phopshoric triamide compound. The phosphoric triamide compound is present in an amount of from about 1 ppm to about 2,500 ppm.

In another embodiment, this invention provides a treated functional particle comprising a functional particle and, based on the weight of the treated functional particle, from about 0.0001 weight percent to about 10 weight percent of an available enzyme inhibitor. Within the scope of one aspect of this invention is a process for the production of a treated functional particle comprising contacting functional particles with from about 0.0001 weight percent to about 10 weight percent of an available enzyme inhibitor based on the weight of the treated functional particle. In a specific embodiment, the treated functional particle is a SAP particle.

The present invention also provides for absorbent structures containing a treated fiber comprising fiber and based on the weight of the treated fiber from about 0.0001 weight percent to about 10 weight percent of an available enzyme inhibitor, a binder, and optionally, functional particles. In certain embodiments, the structure is an airlaid nonwoven structure.

The present invention also provides for a sprayer containing a mechanical or aerosol sprayer, and spray solution containing from about 0.0001 weight percent to about 10 weight percent of an available enzyme inhibitor. In one embodiment, the sprayer has a mass of 2.5 kg or less.

### BRIEF DESCRIPTION OF THE DRAWINGS

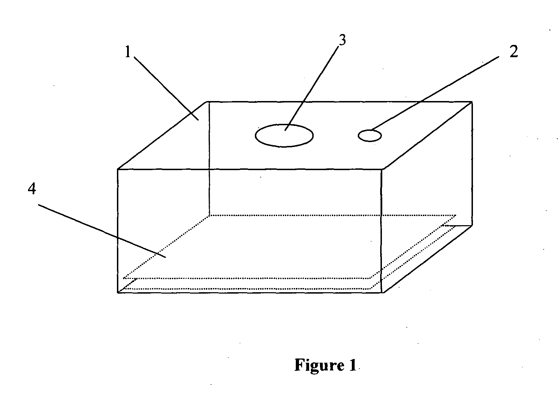

Figure 1 depicts treated VIZORB® 3905 sheets (4) placed in a hermetic plexi-glass container. The container has two outlets in the lid (1): a smaller outlet (2) having a diameter of about 1 cm and a second outlet: (3) having a diameter of about 2.5 cm.

### DETAILED DESCRIPTION

This invention provides a treated fiber, which can be used in personal care articles which, when in use, are insulted by human exudates including urine, but, which, nevertheless, by means of the treatment to the fiber, suppress the formation of objectionable odors which would otherwise occur. The treated fiber of this invention contains one or more enzyme inhibitors.

As used herein, regarding the term "treated fiber", it is not necessary that the enzyme inhibitor compounds chemically bond with the fibers, although it is preferred that the compound remain associated in close proximity with the fibers, by coating, adhering, precipitation, or any other mechanism such that it is not dislodged from the fibers during normal handling of the fibers, absorbent core or absorbent article before contact with liquid or other material. For convenience, the association between the fiber and the compound discussed above may be referred to as the "bond," and the compound may be said to be bound to the fiber.

As used herein, the term "IC₅₀" means the inhibitory concentration (e.g., a micromolar concentration, µM) of a substance (inhibitor) which reduces the rate of substrate cleavage by an enzyme by 50%, as measured by the standard in vitro enzyme activity assays described below. The IC₅₀ is calculated according to the equation IC₅₀=[I]/[(v/vi)-1], where [I] is the inhibitor concentration tested, v is the rate of substrate cleavage in the absence of the inhibitor, and vi is the rate of substrate cleavage in the presence of the inhibitor. As described further below, the IC₅₀ of an enzyme inhibitor according to the invention may be measured by a Purified Enzyme method.

An enzyme inhibitor is effective and available if it inhibits the activity of an enzyme. As used herein, the term "available" means that the enzyme inhibitor on the treated fibers, or other substrate, of this invention is sufficient to cause a reduction in the concentration of generated odor causing agent to about 50 percent or less relative to the untreated control fiber or substrate, two hours after identical insults to the treated fiber, or other substrate, and the control fiber or substrate. Desirably, the reduction is to about 25 percent or less, more desirably, it is to about 10 percent or less, and preferably, it is to about 5 percent or less. It is understood that the size of the sample of treated fibers or other substrate and the control must sized appropriately in comparison to the size of the insult so that the results of this test are on scale, that is, the insulting material must be capable of producing the odor through enzyme activity on the material, and the size should not be so large relative to the size of the tested material that both the test material and the control are swamped.

As used herein, the term "inhibitory effectiveness" means 100 percent minus the percentage reduction in the concentration of generated odor causing agent from the treated fiber, or other substrate, relative to the untreated control fiber or substrate, two hours after identical insults to the treated fiber, or other substrate, and the control fiber or substrate. Thus, a treated fiber or other substrate of this invention with an available enzyme inhibitor has an inhibitory effectiveness of about 50 percent or greater, desirably, of about 75 percent or greater, more desirably, of about 90 percent or greater, and, preferably, of about 95 percent or greater.

The term "about" or "approximately" means within an acceptable error range for the particular value as determined by one of ordinary skill in the art, which will depend in part on how the value is measured or determined, i.e. the limitations of the measurement system. For example, "about" can mean within 1 or more than 1 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

### Enzyme Inhibitors

Inhibitors of enzyme activity are well known and are typically classified as competitive inhibitors, which compete with the substrate for binding at the active site on the enzyme, and non-competitive inhibitors, which bind to a site other than the active site to inactivate the enzyme. Many enzymes, such as metalloproteases, are inhibited by substances that bind with a metal group on the enzyme. Chelating agents are effective inhibitors of other enzymes that, for activation, require the presence of metal ions, such as the ions of calcium, cobalt, copper, magnesium, manganese, sodium, potassium, or zinc. Since enzymes are proteins, antibodies raised against specific enzymes are also effective enzyme inhibitors.

Enzyme inhibitors useful in the treated fiber described herein will typically have an IC₅₀ value of not more than about 500 µM, more typically not more than about 250 µM, still more typically not more than about 100 µM, and still more typically not more than about 50 µM. It will be understood that certain enzyme inhibitors, such as, for example, EDTA, will have higher IC₅₀ values but will still be useful for the preparation of the treated fiber described herein. For materials for which the molecular weight cannot be determined, such materials will typically reduce enzyme activity by at least 50% at a concentration in the treated fiber of about 5 percent by weight or less, based on the weight of the treated fiber. Representative methods for measuring enzyme inhibitory activity are discussed below.

Without limitation, any type of enzyme inhibitor may be employed in the treated fiber of the present invention, including any naturally occurring inhibitor of plant, microbial and/or animal origin, including human, and synthetically manufactured chemical inhibitors. The enzyme inhibitors are preferably in soluble form. The enzyme inhibitors may be hydrophilic or hydrophobic in nature and may thus be water soluble or soluble in a hydrophobic vehicle. The enzyme inhibitors are preferably present in the treated fiber composition in a concentration of from about 0.0001 % to about 10% by weight, typically about 0.001 % to about 2.5%, more typically about 0.01 % to about 1%, and occasionally about 0.1 % to about 0.5%. Because of the variety of enzyme inhibitors employed in the invention, the effective concentration of each inhibitor must be separately determined, as would be known to those skilled in the art. These ranges generally apply for various other embodiments including the nonwoven, the functional particle and the spray solution.

Just as the concentration of the enzyme inhibitor necessary to achieve a given level of inhibitory effectiveness varies from one inhibitor to another, for an inhibitor of multiple enzymes the inhibitory effectiveness may vary depending on the type of insult. For these reasons, it is useful to discuss ranges for the various classes of enzyme inhibitors.

For various metal ions, it is desirable that the metal ion be present and available in an amount from about 1 ppm (parts per million by weight) to about 100,000 ppm, more desirably from about 10 ppm to about 10,000 ppm and still more desirably, from about 100 ppm to about 1,000 ppm.

In various alternative embodiments, it is desirable that the metal ion be present and available such that the metal salt has a metal ion content of from about 0.0005 weight percent to about 5 weight percent based on the weight of the treated fiber, more desirably, from about 0.0005 weight percent to about 3 weight percent, still more desirably, from about 0.0005 weight percent to about 2 weight percent, preferably, from about 0.001 weight percent to about 2 weight percent, and more preferably, from about 0.01 weight percent to about 2 weight percent.

For various enzyme inhibitors, especially salts, the metal ion of the salt may be replaced with ammonium ion, for example, ammonium thiosulfate. In this regard, the terms "metal salt" and "metal ion" as used herein should be read to include ammonium ion.

For other enzyme inhibitors, for example, phosphoric triamide inhibitors, it is desirable that the compound be present and active in an amount of from abut 1 ppm to about 2,500 ppm, more desirably, in an amount of from about 10 ppm to about 1,000 ppm, still more desirably, in an amount of from about 10 ppm to about 500 ppm.

The enzyme inhibitors may be employed singly or as a mixture of enzyme inhibitors such as a "cocktail" of inhibitors in a single embodiment. Moreover, different enzyme inhibitors may be employed in different treated fiber compositions which may be components of a mixture of treated fibers, or a mixture of treated and untreated fibers. Similarly, mixtures of various enzyme inhibitors may be used to treat the nonwovens and functional particles of this invention and be present in a spray solution.

Because of the wide diversity of enzymes present in urine, feces and other body exudates, it is reasonably predictable that materials such as those described below which inhibit certain classes of enzymes, such as, for example, proteases, may also inhibit enzymes that cleave substrates other than those specified, such as proteins and peptides. Hence, inhibitors that inhibit proteases may also inhibit lipases and other esterases, amylases and/or ureases and vice versa.

Inhibitors of enzymes and/or coenzymes most frequently found in urine, feces or other body exudates are preferred in the treated fiber compositions of this invention. Thus, the enzyme inhibitors are preferably inhibitors of proteolytic enzymes such as trypsin, chymotrypsin, aminopeptidase and elastase; lipases; bile salts; amylases; and/or ureases.

Exemplary suitable inhibitors of proteases for use in the invention that are believed to inhibit the type of protease (indicated in parentheses below) include, but are not limited to, soybean trypsin inhibitor and other plant-derived trypsin inhibitors such as lima bean protease inhibitor, corn protease inhibitor and the like; Bowman-Birk inhibitor, serine, trypsin-like protease inhibitor; pancreatic trypsin inhibitor, such as bovine pancreatic basic trypsin inhibitor and other animal-derived pancreatic trypsin inhibitors; egg white trypsin inhibitor, serine, trypsin-like protease inhibitor; ovomucoids containing ovoinhibitors such as from chicken or turkey egg white, trypsin and chymotrypsin inhibitors; chymostatin, serine, chymotrypsin-like protease inhibitor; aprotinin, serine protease inhibitor; leupeptin and its analogs such as propionyl-leupeptin, N-α-t-BOC-deacetylleupeptin, serine and cysteine protease inhibitor; bestatin and its analogs such as epibestatin and nitrobestatin, aminopeptidase metalloprotease inhibitor; amastatin and its analogs such as epiamastatin, aminopeptidase inhibitor; antipain (trypsin inhibitor); antithrombin III (serine protease inhibitor); hirudin (thrombin-like serine protease inhibitor); cystatin (egg white cysteine protease inhibitor); E-64 (trans-epoxysuccinyl-L-leucylamido-(4-guanidino)-butane) and its analogs (cysteine protease inhibitor); α₂ -macroglobulin (universal endoprotease inhibitor); α₁-antitrypsin (trypsin inhibitor); pepstatin and its analogs such as acetyl pepstatin, pepstatin A, NIe-Sta-Ala-Sta (aspartyl protease inhibitor); apstatin (aminopeptidase P inhibitor); (2R)-2-mercaptomethyl-4-methylpentanoyl-b-(2-naphthyl)-Ala-Ala amide (matrix metalloprotease inhibitor); (2R)-2-mercaptomethyl-4-methylpentanoyl-Phe-Ala amide (matrix metalloprotease inhibitor); N-acetyl-Leu-Leu-methioninal (calpain inhibitor); N-acetyl-Leu-Leu-norleucinal (calpain inhibitor); p-aminobenzyol-Gly-Pro-D-Leu-D-Ala hydroxamic acid (matrix metalloprotease inhibitor); 2(R)-[N-(4-methoxyphenylsulfonyl)-N-(3-pyridylmethyl)amino]-3-methylbutano -hydroxamic acid (metalloprotease inhibitor); L-1-chloro-3-[4-tosylamido]-7-amino-2-heptanone-HCl (TLCK), L-1-chloro-3-[4-tosylamido]-4-phenyl-2-butanone (TPCK), tranexamic acid, glycyrrhizic acid, 18-β-glycyrrhetinic acid, and corresponding salts, stearylglycyrrhetinate, colloidal oat extracts, elhibin, zinc salts, iodoacetate, phenylmethylsulfonyl fluoride, phosphoramidon, 4-(2-aminoethyl)-benzenesulfonylfluoride HCl, 3,4-dichloroiso-coumarin, quercetin, and the like, and mixtures thereof.

Chelating agents have also been found to be useful as inhibitors of both proteases and ureases at a concentration of about 0.1 % to about 2%. Exemplary chelating agents are phytic acid, nitrilotriacetic acid, EDTA, diethylene triamino pentacetic acid, hyroxyethyl ethylene diamine triacetic acid, and the corresponding salts, as disclosed in U.S. Pat. No. 5,091,193 (to Enjolras), the disclosure of which is hereby incorporated by reference in its entirety.

Among preferred protease inhibitors for use in the absorbent articles of the invention are compounds that exhibit inhibitory activity that is not necessarily restricted to a single class of proteases. Such compounds include, but are not limited to, hexamidine and its salts; pentamidine and its salts; benzamidine and its salts and derivatives, p-aminobenzamidine and its salts and derivatives; and guanidinobenzoic acid and its salts and derivatives such as those disclosed in U.S. Patent No. 5,376,655 (to Imaki et al.), the disclosure of which is hereby incorporated herein by reference in its entirety. Other preferred protease inhibitors include polymer derivatives of guanidinobenzoic acid disclosed and made in U.S. Patent No. 6,066,673, the disclosure of which is hereby incorporated herein by reference in its entirety.

Protease inhibitors that are preferred in the practice of the invention are soybean trypsin inhibitor, aprotinin, hexamidine, hexamidine diisethionate, p-aminobenzamidine, leupeptin, pepstatin A, chymostatin and polymer derivatives of guanidinobenzoic acid, disclosed in U.S. Patent No. 6,066,673. Particularly preferred protease inhibitors are soybean trypsin inhibitor, hexamidine, p-aminobenzamidine and the foregoing polymer derivatives of guanidinobenzoic acid.

Ureases are known to be inhibited in the presence of trace amounts of heavy metal ions, such as those of silver, copper, and the like. Thus, trace amounts, as little as 0.001% or less, of salts of these metals are useful as urease inhibitors. Other exemplary inhibitors of urease activity include, but are not limited to, acetyl hydroxamic acid and its derivatives, such as cinnamoyl hydroxamic acid and other alkyl hydroxamic acids, corresponding salts and derivatives; phosphoramidate and its derivatives. Such compounds are competitive inhibitors of urease at a concentration of about 2 micromolar (µM). As noted below, chelating agents have also been found to be useful as inhibitors of both proteases and ureases at a concentration of about 0.1 % to about 2%. Exemplary chelating agents are phytic acid, nitrilotriacetic acid, ethylenediamine tetraacetic acid, EDTA, diethylene triamino pentacetic acid, hyroxyethyl ethylene diamine triacetic acid, and the corresponding salts, disclosed in U.S. Patent No. 5,091,193, hereby incorporated herein by reference in its entirety. Other urease inhibiting compounds are disclosed in U.S. Patent No. 3,935,862 (to Kraskin), the disclosure of which is hereby incorporated herein by reference in its entirety, and include amino acid compounds, such as hydroxyalkylamino acids, sulfhydryl amino acids, aminosulfonic acids, aminophosphonic acid compounds and ether amino acids such as methoxyethyliminodiacetic acid, ethylene-bis-(oxypropylaminodiacetic acid), ethylene-bis-(oxyethyliminodiacetic acid), amino-methyl phosphonic acid (N,N-diacetic acid), and the like, and aminopolycarboxylic acid compounds, including acids and salts diethylenetriaminepentaacetic acid (DTPA), N-hydroxy-ethylethylenediaminetriacetic acid (HEDTA), and the like.

Other suitable inhibitors of urease are disclosed in U.S. Patent No. 5,409,903 (to Polak et al.), the disclosure of which is hereby incorporated herein by reference in its entirety. This patent discloses dibasic magnesium phosphate, dialdehyde polysaccharides and zeolite, used alone in combination with each other or with the calcium compounds, calcium acetate, calcium chloride, calcium gluconate and calcium lactate as well as the magnesium compounds, magnesium chloride and magnesium citrate, for inhibition of ureases.

Suitable lipase inhibitors include, but are not limited to, water soluble salts of metals, such as cadmium, cobalt, copper, iron, molybdenum, silver, lanthanum, tin and zinc. Exemplary lipase inhibiting compounds are disclosed in U.S. Patent No. 4,556,560, hereby incorporated herein by reference in its entirety, and include zinc chloride, zinc acetate, zinc nitrate trihydrate, zinc nitrate hexahydrate, zinc sulfate, zinc sulfate heptahydrate, zinc sulfate hexahydrate, iron(II) chloride, iron(II) chloride tetrahydrate, iron(III) chloride, iron(III) chloride monohydrate, iron(III) chloride hexahydrate, iron(II) lactate, iron(III) lactate, iron(III) malate, iron(II) nitrate, iron(III) nitrate hexahydrate, iron(III) nitrate 9H₂O, iron(II) sulfate and its hydrates, iron(III) sulfate and its hydrates, copper sulfate pentahydrate, tin chloride, cobalt chloride and lanthanum chloride, zinc salts of both saturated and unsaturated monocarboxylic acids having about 6 to about 12 carbon atoms, block copolymers of propylene oxide and ethylene oxide, such as those marketed as Pluronic® and Tetronic® by BASF Corp., glycerol triesters of fatty acids having from about 2 to about 20 carbons such as triacetin, and the like. Other useful lipase inhibitors are disclosed in U.S. Patent No. 5,091,193, hereby incorporated herein by reference in its entirety, and include esters of fatty alcohols, such as saturated or unsaturated, linear or branched alkyl acetate, lactate or propionate containing 10 to 20 carbon atoms; saturated or unsaturated, linear or branched zinc salts of fatty acids of 2 to 22 carbon atoms, such as those formed with propionic acid isobutyric acid, caproic acid, undecylenic acid, and the like; zinc salts of aminated acylated acids, such as propionylcysteine, propionyl-hydroxyproline or caproylcysteine, and the like. Lipase inhibitors, such as the foregoing, have been found to be useful at a concentration of about 0.01 % to about 10%.

Still other useful lipase inhibitors, disclosed in pending Patent Application EP 0 922 456 A1 (to Palumbo et al.), the disclosure of which is hereby incorporated herein by reference in its entirety, include specific ester compounds that act as a substitute substrate for fecal lipases and thereby are competitive lipase inhibitors. These esters have the formulas (I) or (II): 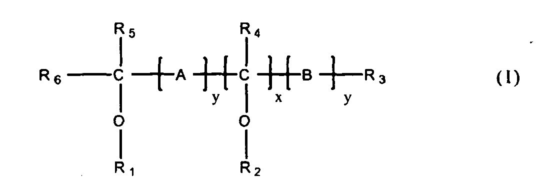
or 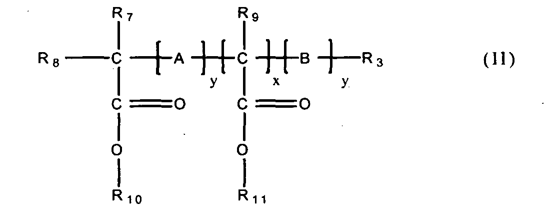
wherein R₁ and each R₂ independently are an acyl group with from 2 to 22 carbon atoms, or an alkyl, alkenyl, arylalkyl, hydroxyalkyl group with from 1 to 24 carbon atoms or hydrogen, whereby at least one of R₁ and R₂ is such an acyl group, R₃, R₄, R₅, R₆, R₇, R₈, and R₉ are independently an alkyl, alkenyl, arylalkyl, hydroxyalkyl, alkoxy groups of from 1 to 24 carbon atoms, hydroxy group or hydrogen; R₁₀ and R₁₁ are independently an alkyl, alkenyl, arylalkyl, hydroxyalkyl, alkoxy groups of from 2 to 24 carbon atoms, hydroxy group or hydrogen; A and B are independently a C₁ -C₆ linear or branched alkylene, alkenylene, alkoxylene, hydroxy-alkylene groups; the values of x are independently from 0 to 15; the values of y are independently 0 or 1, with the proviso that when x=2 and y=0, at least one R₂ is an alkyl, alkenyl, arylalkyl, hydroxyalkyl group with from 1 to 24 carbon atoms or hydrogen.

Still further examples of lipase inhibitors are those disclosed in U.S. Patent No. 5,643,874, hereby incorporated herein by reference in its entirety, which include: (2S,3S,5S)-5-[(S)-2-formamido-4-methyl-valeryloxy]-2-hexyl-3-hydroxy-hexad ecanoic 1,3 acid lactone, also known as tetrahydrolipstatin; (2S,3S,5S,7Z,10Z)-5-[(S)-2-formamido-4-methyl-valeryloxy]-2-hexyl-3-hydroxy-7,10-hexade cadienoic 1,3 acid lactone, also known as lipstatin; 1-(trans-4-isobutylcyclohexyl)-2-(phenylsulfonyloxy)ethanone, also known as FL-386; 4-methylpiperidine-1-carboxylic acid 4-phenoxyphenyl ester, also known as WAY-121898; N-[3-chloro-4-(trifluoromethyl)phenyl-]N'-[3-(trifluoromethyl)-phenyl]urea, also known as BAY-N-3176; N-formyl-L-valine-(S)-1-[[(2S, 3S)-3-hexyl-4-oxo-2-oxetanyl]methyl]hexyl ester, also known as valilactone; (2S,3S,5S,7Z, 10Z)-5-[(S)-2-acetamido-3-carbamoylpropionyloxy]-2-hexyl-3-hydroxy-7,10-he xadecadienoic lactone, also known as esterastin; (3S,4S)-4-[(1S,5R,7S,8R,9R,E)-8-hydroxy 1,3,5,7,9-pentamethyl-6-oxo-3-undecenyl]-3-methyl-2-oxetanone, also known as ebelactone A; (3S,4S)-3-ethyl-4-[(1S,5R,7S,8R,9R,E)-8-hydroxy-1,3,5,7,9-pentamethyl-6-ox o-3-undecenyl]-2-oxetanone, also known as ebelactone B; and 1,6-di(O-(carbamoyl)cyclohexanone oxime)hexane, also known as RHC 80267.

Exemplary inhibitors of bile salts that are coenzymes for lipolytic enzymes and are useful as lipase enzyme inhibitors in the absorbent articles of the invention include, but are not limited to, cationic compounds disclosed in EP Patent Application No. EP 0 922 452 (to Palumbo et al.), the disclosure of which is hereby incorporated herein by reference in its entirety. Such compounds have the formulas (III or IV): 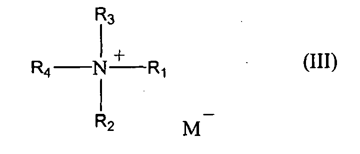
or 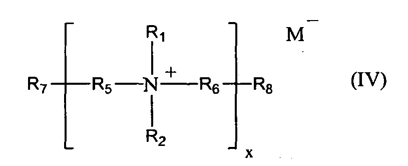
or an amphoteric compound and preferably an acidity source, the amphoteric compound having at its iso-electric point the formula (V): 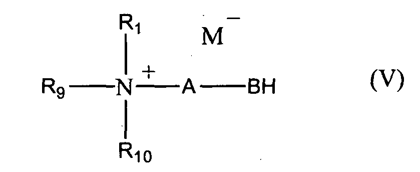
for preparation of a composition for treatment, prevention or reduction of lipolytic dermatitis of the external skin, wherein R₁, R₂, R₃ and R₄ are independently a C₁ -C₂₂ alkyl, alkenyl, aryl, arylalkyl, amidoalkyl, (poly) alkoxy, hydroxyalkyl, or acyl groups, or two or more groups of R₁, R₂, R₃ and R₄ form together one or more ring structures; R₅, R₆ and A are independently a C₁ -C₂₂ alkylene, alkenylene, (poly) alkoxylene, hydroxyalkylene, arylalkylene or amido alkylene groups; R₇ and R₈ are independently a C₁ -C₄ alkyl, alkenyl, alkoxy group or a hydroxy group or hydrogen; R₉ and R₁₀ are independently a C₁ -C₂₂ alkyl, alkenyl, aryl, arylalkyl, amidoalkyl, (poly) alkoxy, hydroxyalkyl, or acyl groups, or two or more of the groups R₁, R₉ and R₁₀ form together one or more ring structures; BH is a proton donating group; x is from 2 to 4; and M- is a counter ion.

Another exemplary suitable bile salt inhibitor is cholestyramine, described in a publication by C. Michael White et al., entitled *Cholestyramine Ointment to Treat Buttocks Rash and Anal Excoriation in an Infant,* The Annals of Pharmacotherapy 30: 954-956, September 1996.

Derivatives of p-guanidinobenzoic acid, especially esters of p-guanidinobenzoic acid, have been described as inhibitors of esterases. Such inhibitors are useful in the skin care compositions of the absorbent articles of the invention, and are disclosed in U.S. Patent No. 5,376,655, the disclosure of which is hereby incorporated herein by reference in its entirety.

Suitable amylase inhibitors and/or glucosidase amylase inhibitors include those disclosed in U.S. Patent No. 5,643,874, hereby incorporated herein by reference in its entirety, and include O-4,6-dideoxy-4-[[[1S-(1α,4α,5β,6α)]-4,5,6-trihydroxy-3-(hydroxymethyl)-2-cyclohexen-1-yl]amino]-α-D-glucopyranosyl-(1→4)O-α-D-glucopyranosyl-(1→4)-D-glucose, also known as acarbose; 2(S),3(R),4(S),5(S)-tetrahydroxy-N-[2-hydroxy-1-(hydroxymethyl)-ethyl]-5-(hydroxymethyl)-1(S)-cyclohexamine, also known as voglibose;1,5-dideoxy-1,5-[(2-hydroxyethyl)imino]-D-glucitol, also known as miglitol; 1,5-dideoxy-1,5-[2-(4-ethoxycarbonylphenoxy)-ethylimino]-D-glucitol, also known as emiglitate; 2,6-dideoxy-2,6-imino-7-(β-D-glucopyranosyl)-D-glycero-L-guloheptitol , also known as MDL-25637; 1,5-dideoxy-1,5-(6-deoxy-1-O-methyl-a-D-glucopyranos-6-ylimino)-D-gucitol, also known as camiglibose; 1,5,9,11,14-pentahydroxy-3-methyl-8,13-dioxo-5,6,8,13-tetrahydrobenzo[a]-n aphthacene-2-carboxylic acid, also known pradimicin Q; also known as adiposine; and 1,2-dideoxy-2-[2(S),3(S),4(R)-trihydroxy-5-(hydroxymethyl)-5-cyclohexen-1(S)-ylamino]-L-glucopyranose, also known as salbostatin. Other suitable amylase inhibitors include tendamistat, trestatins, and those derived from plants, especially from wheat, rice, maize, barley and other cereal grains, beans, and seaweed.

In one embodiment of this invention, treated fibers are prepared by contacting the fibers with, preferably, an aqueous solution of the antimicrobial and/or urease inhibitor, preferably transition metal ion. Suitable sources of the transition metal ions are their soluble salts. The preferred salts are silver, copper, zinc, ferric, and aluminum salts, more preferably zinc. It is also desirable that the anion provide some benefit, that is, that the anion can have the ability to provide urease inhibition, such as borate, phytate, etc. Suitable examples are silver chlorate, silver nitrate, mercury acetate, mercury chloride, mercury nitrate, copper metaborate, copper bromate, copper bromide, copper chloride, copper dichromate, copper nitrate, copper salicylate, copper sulfate, zinc acetate, zinc borate, zinc phytate, zinc bromate, zinc bromide, zinc chlorate, zinc chloride, zinc sulfate, cadmium acetate, cadmium borate, cadmium bromide, cadmium chlorate, cadmium chloride, cadmium formate, cadmium iodate, cadmium iodide, cadmium permanganate, cadmium nitrate, cadmium sulfate, and gold chloride. Other salts that have been disclosed as having urease inhibition properties include ferric and aluminum salts, especially the nitrates, and bismuth salts. Zinc salts are preferred, especially zinc sulfate and zinc chloride.

Silver salts and mercury salts are very effective but are also toxic and expensive and are therefore used at levels ranging from about 50 ppm to about 500 ppm, preferably from about 100 ppm to about 300 ppm. Copper salts, zinc salts and cadmium salts are most effectively used at levels ranging from about 1 ppm to about 7500 ppm, desirably from about 10 ppm to about 5000 ppm, more desirably from about 50 ppm to about 3000 ppm, preferably at levels from about 100 ppm to about 2000 ppm, more preferably from about 150 ppm to about 1000 ppm. Gold salts are effective and substantially less toxic than silver or mercury.

The preferred metallic salt, preferably water-soluble zinc salts, can be added to the solution used to prepare the litter of the present invention. A water-soluble metallic salt can be used as an odor control agent. A water-soluble metallic salt can be present in the freshening composition of the present invention to absorb amine and sulfur-containing compounds. Furthermore, they usually do not contribute an odor of their own. Preferably the water-soluble metallic salts are selected from the group consisting of copper salts, zinc salts, and mixtures thereof.

The preferred zinc salts have been used most often for their ability to ameliorate malodor, e.g., in mouth wash products, as disclosed in U.S. Patent No. 4,325,939 and U.S. Patent No. 4,469,674 hereby incorporated herein by reference in their entirety. U.S. Patent No. 3,172,817 (to Leupold, et al.), hereby incorporated herein by reference in its entirety discloses deodorizing compositions containing slightly water-soluble salts of an acyl-acetone with a polyvalent metal, including copper and zinc salts. The zinc salts are preferably water soluble, and therefore the solutions herein should not be so alkaline so as to avoid formation of zinc oxide, which is much less soluble.

Examples of preferred water-soluble zinc salts are zinc chloride, zinc gluconate, zinc lactate, zinc maleate, zinc salicylate, zinc sulfate, etc. Highly-ionized and soluble zinc salts such as zinc chloride, provide the best source of zinc ions. Examples of preferred copper salts are copper chloride and copper gluconate. Preferred metallic salts are zinc chloride and copper chloride.

Metallic salts are added to the fiber composition of the present invention typically at a level of from about 0.001 % to about 2%, preferably from about 0.01% to about 1%, more preferably from about 0.05% to about 0.5%, by weight of the composition. When zinc salts are used as the metallic salt, it is preferable that the pH of the solution is adjusted to less than about 7, more preferably less than about 6, most preferably, less than about 5, in order to keep the solution clear.

In another embodiment of this invention, treated fibers and nonwoven materials are produced by contacting them with an effective amount, desirably, a urease inhibiting effective amount, of one or more phosphoric triamide compounds of the formula (VI): 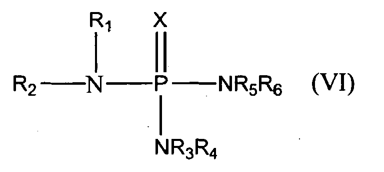
wherein:
X is oxygen or sulfur;
R₁ is alkyl, cycloalkenyl, aralkyl, alkenyl, alkynyl, or cycloalkyl;
R₂ is R₁, hydrogen, or R₁ and R₂ together may form an alkylene or alkenylene chain which may optionally include one or more heteroatoms of divalent oxygen, nitrogen or sulfur completing a 4, 5, 6, 7, or 8 membered ring system; and
R₃, R₄, R₅ and R₆ are individually hydrogen or alkyl having 1 to about 4 carbon atoms. In the present specification and claims, the term "phosphoric triamide compounds" is used to refer to these compounds.

The phosphoric triamide compounds which are employed as urease inhibitors in the composition and method of this invention are those of the formula (VII): 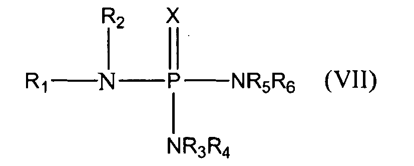
wherein:
X is sulfur or oxygen;
R₁ is alkyl, alkenyl, alkynyl, cycloalkyl, aralkyl or cycloalkenyl;
R₂ is R₁, hydrogen, or R₁ and R₂ together may form an alkylene or alkenylene chain optionally containing one or more heteroatoms of oxygen, sulfur or nitrogen completing a 3, 4, 5, 6, 7 or 8 membered ring system; and
R₃, R₄, R₅ and R₆ are the same or different and are individually hydrogen or alkyl having from 1 to about 4 carbon.

Illustrative of permissible R₁ substituents are tert-butyl, neopentyl, tetramethylbutyl, methyl, pentyl, hexyl, heptyl, sec-octyl, dodecyl, sec-butyl, ethyl, propyl, oleyl, isopropyl, butyl, propargyl, isobutyl, isopentyl, sec-pentyl, hexyl, sec-heptyl, heptyl, octyl, cyclopropyl, cyclobutyl, propenyl, pentenyl, sec-hexyl, cyclohexyl, hexenyl, cyclopentenyl, allyl, sec-isohexyl, 2-phenylethyl, 2-naphthylethyl, cyclohexenyl, benzyl and the like.

Exemplary of useful R₂ substituents are hydrogen, methyl, ethyl, propyl; isopropyl, butyl, isobutyl, pentyl, neopentyl, hexyl, 2-butene, ethylene, 3-butene, 2-propene, acetylene, cyclopropyl, cyclobutyl, cyclopentyl, cyclohexyl, and the like.

Permissible R₃, R₄, R₅ and R₆ substituents include hydrogen, methyl, ethyl, propyl, isopropyl, butyl, isobutyl and the like.

The following compounds are illustrative of phosphoric triamide compounds within the purview of the above structural formula which can be prepared simply by selecting appropriate reactants for use in the procedures described below and which can be employed in the practice of this invention.
N-(sec-pentyl)phosphoric triamide
N-(sec-hexyl)phosphoric triamide
N-(sec-isohexyl)phosphoric triamide
N-(n-heptyl)phosphoric triamide
N-(n-propyl)phosphoric triamide
N-(n-butyl)-N-methylphosphoric triamide
N-(sec-butyl)-N-methylphosphoric triamide
N-(sec-pentyl)-N-ethylphosphoric triamide
N-(iso-propyl)-N-methylphosphoric triamide
N,N-diisopropylphosphoric triamide
N-(sec-isohexyl)phosphoric triamide
N-(n-butyl)-N'-methylphosphoric triamide
N-(ethyl)-N'-isopropylphosphoric triamide
N-butyl-N'-ethyl-N"-propylphosphoric triamide
N-3-butenylphosphoric triamide
N-propargylphosphoric triamide
N-neopentylphosphoric triamide
N-hexylphosphoric triamide
N,N-bis-(sec-butyl)phosphoric triamide
N-methyl-N-propylphosphoric triamide
N-(2-cyclohexenylphosphoric triamide
N-cyclopropylphosphoric triamide
N-cyclopentenylphosphoric triamide
N-cyclobutylphosphoric triamide
N-cyclopentylphosphoric triamide
N,N'-dimethyl-N-propylphosphoric triamide
N-cyclopentyl-N-benzylphosphoric triamide
N-[2-(1'-naphthyl)ethyl]phosphoric triamide
N-cyclopropyl-N-methylphosphoric triamide
N-ethyl-N-methylphosphoric triamide
N,N-dipropylphosphoric triamide
N-(3-benzylpropyl)phosphoric triamide
N-isobutyl-N-(2-butenyl)phosphoric triamide
N-methyl-N'-benzylphosphoric triamide
N-ethyl-N-(3-phenylpropyl)phosphoric triamide
N-2-(3-pyridyl)ethyl phosphoric triamide
N-(2-thienylethyl)phosphoric triamide
N-(3-phenylpropyl)phosphoric triamide
N-(4-phenylbutyl)phosphoric triamide
N-butyl-N-isopropylphosphoric triamide
N-methyl-N-[2-(1-naphthyl)ethyl]phosphoric triamide
N-diaminophosphinylazolidine
N-diaminophosphinyloxathioazolidine
N-cyclopropylphosphoric triamide
N-(diaminophosphinyl)oxazolidine
N-(2-phenylethyl)phosphoric triamide
N-(diaminophosphinyl)aziridine
N-(diaminophosphinyl)pyrrolidine
N-(diaminophosphinyl)thiazine
N-(2-thienyl)methylphosphoric triamide
N-(diaminophosphinyl)oxathiazine
N-(diaminophosphinyl)oxazine
N-(diaminophosphinyl)triazine
N-(diaminophosphinyl)azine
N-[2-(2'-naphthyl)ethyl]phosphoric triamide
N-5-(1,2,4-thiadiazole)phosphoric triamide
N-5-(3-trichloromethyl-1,2,4-thiadiazole)phosphoric triamide
N-cyclohexyl-N-methylphosphoric triamide
N-methyl-N-propylphosphoric triamide
N-(diaminophosphinyl)morpholine
N-(diaminophosphinyl)thiomorpholine
N-(diaminophosphinyl)piperazine
N-(diaminophosphinyl)pyrimidine
N-methyl-N-(3-phenylpropyl)phosphoric triamide
N-(diaminophosphinyl)pyrrole
N-(diaminophosphinyl)pyrazole
N-(diaminophosphinyl)imidazole
N-(diaminophosphinyl)-1,2,3-triazole
N-(diaminophosphinyl)-1,2,4-triazole
N-(diaminophosphinyl)tetrazole
N-(diaminophosphinyl)indole
N-(diaminophosphinyl)benzotriazole
N-(diaminophosphinyl)benzoimidazole
N-(sec-pentyl)thiophosphoric triamide
N-(sec-hexyl)thiophosphoric triamide
N-(sec-isohexyl)thiophosphoric triamide
N-(n-heptyl)thiophosphoric triamide
N-(n-propyl)thiophosphoric triamide
N-(n-butyl)-N-thiophosphoric triamide
N-(sec-butyl)-N-methylthiophosphoric triamide
N-(sec-pentyl)-N-ethylthiophosphoric triamide
N-(iso-propyl)-N-methylthiophosphoric triamide
N,N-di(n-dodecyl)thiophosphoric triamide
N-(sec-isohexyl)thiophosphoric triamide
N-(n-butyl)-N'-methylthiophosphoric triamide
N-(ethyl)-N'-isopropylthiophosphoric triamide
N-butyl-N'-ethyl-N"-propylthiophosphoric triamide
N-3-butenylthiophosphoric triamide
N-propargylthiophosphoric triamide
N-neopentylthiophosphoric triamide
N-[n-(5-hexynyl)]thiophosphoric triamide
N-octylthiophosphoric triamide
N-methyl-N-propylthiophosphoric triamide
N-(2-phenethyl)thiophosphoric triamide
N-(2-cyclohexenyl)thiophosphoric triamide
N-cyclopropylthiophosphoric triamide
N-cyclopentenylthiophosphoric triamide
N-cyclobutylthiophosphoric triamide
N-cyclopentylthiophosphoric triamide
N,N'-dimethyl-N-propylthiophosphoric triamide
N-[2-(1'-naphthyl)ethyl]thiophosphoric triamide
N-cyclopropyl-N-methylthiophosphoric triamide
N-ethyl-N-methylthiophosphoric triamide
N,N-dipropylthiophosphoric triamide
N-(3-benzylpropyl)thiophosphoric triamide
N-isobutyl-N-(2-butenyl)thiophosphoric triamide
N-methyl-N-(1-naphthyl)thiophosphoric triamide
N-ethyl-N-(3-phenylpropyl)thiophosphoric triamide
N-2-(3-pyridyl)ethylthiophosphoric triamide
N-(2-thienylethyl)thiophosphoric triamide
N-(3-phenylpropyl)thiophosphoric triamide
N-(4-phenylbutyl)thiophosphoric triamide
N-isopropylthiophosphoric triamide
N-methyl-N-[2-(1-naphthyl)ethyl]thiophosphoric triamide
N-diaminothiophosphinylazolidine
N-diaminothiophosphinyloxathioazolidine
N-(diaminothiophosphinyl)oxazolidine
N-(2-phenylethyl)thiophosphoric triamide
N-(diaminothiophosphinyl)aziridine
N-(diaminothiophosphinyl)oxathiazolidine
N-(diaminothiophosphinyl)thiazine
N-(2-thienyl)methylthiophosphoric triamide
N-(diaminothiophosphinyl)oxathiazine
N-(diaminothiophosphinyl)oxazine
N,N-dimethylthiophosphoric triamide
N-(diaminothiophosphinyl)triazine
N-(diaminothiophosphinyl)azine
N-[2-(2'-naphthyl)ethyl]thiophosphoric triamide
N-cyclohexyl-N-methylthiophosphoric triamide
N-methyl-N-propylthiophosphoric triamide
N-5-(1,2,4-thiadiazole)thiophosphoric triamide
N-5-(3-trichloromethyl-1,2,4-thiadiazole)thiophosphoric triamide
N-(diaminothiophosphinyl)morpholine
N-(diaminothiophosphinyl)thiomorpholine
N-(diaminothiophosphinyl)piperazine
N-(diaminothiophosphinyl)pyrimidine
N-methyl-N-(3-phenylpropyl)thiophosphoric triamide
N-(diaminothiophosphinyl)pyrrole
N-(diaminothiophosphinyl)pyrazole
N-(diaminothiophosphinyl)imidazole
N-(diaminothiophosphinyl)-1,2,3-triazole
N-(diaminothiophosphinyl)-1,2,4-triazole
N-(diaminothiophosphinyl)tetrazole
N-(diaminothiophosphinyl)indole
N-(diaminothiophosphinyl)benzotriazole
N-(diaminothiophosphinyl)benzoimidazole
Preferred for use in the practice of this invention are phosphoric triamide compounds in which:
X is sulfur or oxygen;
R₁ is alkyl, aralkyl, alkenyl or cycloalkyl;
R₂ is R₁, hydrogen or R₁ and R₂ together may form an alkylene chain completing a 3, 4, 5 or 6 membered ring system; and
R₃, R₄, R₅ and R₆ are the same or different and are individually hydrogen or methyl.

Particularly preferred for use in this invention are phosphoric triamide compounds in which:
X is oxygen or sulfur;
R₁ is branched chain or linear alkyl having from about 1 to about 8 carbon atoms, phenylalkyl wherein the aliphatic moiety includes from 1 to about 4 carbon atoms, cyclobutyl, cyclopropyl, cyclohexyl or cyclopentyl; and
R₂, R₃, R₄, R₅ and R₆ are hydrogen.

Especially efficacious compounds for use in the practice of this invention are N-cyclohexylphosphoric triamide, N,N-dimethylphosphoric triamide, N-benzyl-N-methylphosphoric triamide, N-isopropylphosphoric triamide, N,N-diethylphosphoric triamide, N-ethylphosphoric triamide, N-(n-butyl)phosphoric triamide, N-(n-butyl)thiophosphoric triamide, N-(sec-butyl)phosphoric triamide, N-(n-dodecyl)phosphoric triamide, N,N-diethylthiophosphoric triamide, N-cyclohexyl-N-methylphosphoric triamide, N-(n-octyl)phosphoric triamide, N-allylphosphoric triamide, N-(diaminophosphinyl)piperidine, N-benzyl-N-methylthiophosphoric triamide, N-cyclohexylthiophosphoric triamide, N-(n-hexyl)thiophosphoric triamide, N-(diaminothiophosphinyl)pyrrolidine, N-(sec-butyl)thiophosphoric triamide, N,N-diisopropylthiophosphoric triamide, N-(diaminothiophosphinyl)piperidine, and N,N-di-(n-butyl)thiophosphoric triamide.

Useful compounds can be prepared in accordance with the following reaction scheme: 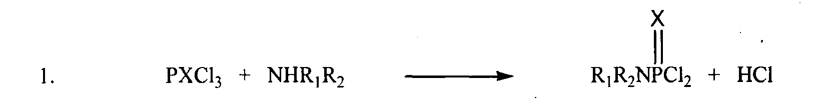 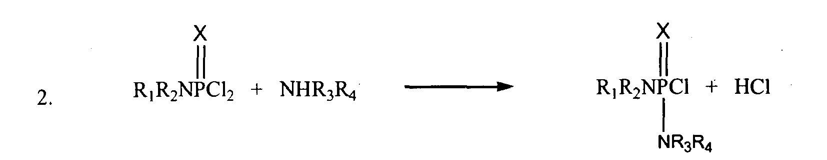 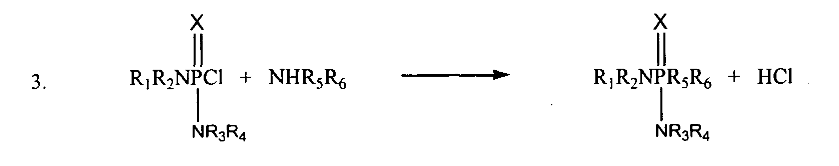
wherein X, R₁, R₂, R₃, R₄, R₅ and R₆ are as described above with the phosphoric triamide compounds. The aforementioned reaction is described in more detail in M. Goehring and K. Niedenzu, Chem. Ber. 89, pp. 1768-1771 (1956) and in references cited therein, and will not be described herein in great detail.

Useful solutes in the present invention include any solute that has odor control properties and does not impart discoloration, e.g., yellowing of the substrate. Examples of such solutes include chelating or sequestering agents (builders). Chelating agents useful in the present invention include the sodium, potassium and ammonium salts of diphosphoric acid, triphosphoric acid, pyrophosphoric acid, orthophosphoric acid, hexametaphosphoric acid, 1-hydroxyethane-1,1-phosphonic acid, diethylenetriamine penta(methylene diphosphonic acid), ethylenediamine tetraacetic acid (EDTA), diethylenetriamine pentaacetic acid (DTPA), N-(hydroxyethyl) ethylenediamine triacetic acid (HEDTA), propylenediamine tetraacetic acid (PDTA), nitrilotriacetic acid (NTA), mellitic acid, 1,2,3,4-cyclopentane tetracarboxylic acid, succinic acid, lauryl succinic acid, oxydisuccinic acid (ODS), carboxymethyloxysuccinic acid, citric acid, lactic acid, tartaric acid, O-carboxymethyltartronic acid, polyacrylic acid, poly(α-hydroxyacrylic acid), poly(tetramethylene-1,2-dicarboxylic acid), poly(4-methoxytetra-methylene-1,2-dicarboxylic acid), acrylic acid/maleic acid copolymer (polycarboxylate), acrylic acid/allyl alcohol copolymer (polycarboxylate), sodium PCA, gluconic acid, glucoheptonic acid, lactobionic acid, maltobionic acid, 1-hydroxyethylidene biphosphate, etidronic acid, amino phosphanates, and mixtures thereof. Na₃-EDTA is one solute that has been identified as being particularly useful.

More specifically, one useful odor control agent was Na₃-EDTA, which is available as Dissolvine® Na₃. Without being bound by theory, it is believed that the high pH of Na₄₋EDTA (pH=about 10) caused oxidation of pulp fibers in a non-woven substrate, which resulted in discoloration, e.g., yellow tint formation. It was found that lowering the pH, whether by using a different salt for of EDTA, e.g., Na₃-EDTA (pH=about 8-9), or by adding acid to the high pH EDTA solution did not result in oxidation of pulp fibers in a non-woven substrate as no discoloration was found.

Na₃-EDTA is also available as Dissolvine® Na₃₋₃₆ as a 36% aqueous solution. Other possible options are Dissolvine® K₃₋₁₂₃-S (K₃-EDTA, 50% solution) and Dissolvine®AM₃₋₄₀ ((NH₄)₃-EDTA, 40% solution). Each is available from Akzo Nobel. Versene®(NH₄)₄EDTA chelating agent (38% aqueous solution) is available form Dow Chemicals.

It is well within the skill of one in the art to make a Na₃-EDTA solution using Na₄₋EDTA as a starting component. Na₄-EDTA is available from many sources including Dow Chemicals (Versene® Powder chelating agent, Versene® Powder A, Versene®220E, Versene® 220 Crystals Chelating agent, Versene®100 XL (38% aqueous solution), Versene®100 EP (38% aqueous solution), Versene® 100 (39% aqueous solution)), Akzo Nobel (Dissolvine® 220-S, Dissolvine® NA-X, Dissolvine® NA, Dissolvine® E-39 (39% aqueous solution), Dissolvine® 100-S (38% aqueous solution)), or BASF (Trilon® B and Trilon® BX).

### Cellulose Fibers

Cellulosic fibrous materials suitable for use in the present invention include softwood fibers and hardwood fibers. See M. J. Kocurek & C. F. B. Stevens, *Pulp and Paper Manufacture--Vol. 1: Properties of Fibrous Raw Materials and Their Preparation for Pulping,* which is hereby incorporated by reference in its entirety, The Joint Textbook Committee of the Paper Industry, 1983, 182 pp. Exemplary, though not exclusive, types of softwood pulps are derived from slash pine, jack pine, radiata pine, loblolly pine, white spruce, lodgepole pine, redwood, and douglas fir. North American southern softwoods and northern softwoods may be used, as well as softwoods from other regions of the world. Hardwood fibers may be obtained from oaks, genus *Quercus*, maples, genus *Acer,* poplars, genus *Populus,* or other commonly pulped species. In general, softwood fibers are preferred due to their longer fiber length as measured by T 233 cm-95, and southern softwood fibers are most preferred due to a higher coarseness as measured by T 234 cm-84, which leads to greater intrinsic fiber strength as measured by breaking load relative to either northern softwood or hardwood fibers.

The fibrous material may be prepared from its natural state by any pulping process including chemical, mechanical, thermomechanical (TMP) and chemithermomechanical pulping (CTMP). These industrial processes are described in detail in R. G. Macdonald & J. N. Franklin, *Pulp and Paper Manufacture in 3 volumes; 2*^{*nd*} *Edition, Volume 1: The pulping of wood, 1969, Volume 2: Control, secondary fiber, structural board, coating, 1969, Volume 3: Papermaking and paperboard making, 1970,* The joint Textbook Committee of the Paper Industry, and in M. J. Kocurek & C. F. B. Stevens, *Pulp and Paper Manufacture, Vol. 1: Properties of Fibrous Raw Materials and Their Preparation for Pulping,* The Joint Textbook Committee of the Paper Industry, 1983, 182 pp., both of which are hereby incorporated by reference in their entirety. Preferably, the fibrous material is prepared by a chemical pulping process, such as a Kraft or sulfite process. In particular the Kraft process is especially preferred. Pulp prepared from a southern softwood by a Kraft process is often called SSK. In a similar manner, southern hardwood, northern softwood and northern hardwood pulps are designated SHK, NSK & NHK, respectively. Bleached pulp, which is fibers that have been delignified to very low levels of lignin, are preferred, although unbleached Kraft fibers may be preferred for some applications due to lower cost, especially if alkaline stability is not an issue. Desirably, the chemically treated cellulose fiber has been derived from a source which is one or more of Southern Softwood Kraft, Northern Softwood Kraft, hardwood, eucalyptus, mechanical, recycle and rayon, preferably Southern Softwood Kraft, Northern Softwood Kraft, or a mixture thereof, more preferably, Southern Softwood Kraft.

Pulp consistency is a pulp-industry specific term which is defined as the bone dry fiber amount divided by the total amount which includes fiber, water, other solids, etc. and multiplied by 100 percent. Therefore, for a slurry of 12 percent consistency, every 100 kilograms of slurry would contain 12 bone dry kilograms of fiber.

The treated fiber of this invention may be treated while the fiber is in individualized form or it may be treated while in the form of a sheet, such as a comminution sheet, or other agglomerated form which then later is comminuted to form individualized fibers. The individualized fibers can be used in the formation of various nonwoven materials and other articles for personal care use. For some applications it may desirable to prepare a nonwoven material and then treat the material.

Various materials, structures and manufacturing processes useful in the practice of this aspect of this invention are disclosed in U.S. Patent Nos. 6,241,713; 6,353,148; 6,171,441; 6,159,335; 5,695,486; 6,344,109; 5,068,079; 5,269,049; 5,693,162; 5,922,163; 6,007,653; 6,355,079; 6,403,857, 6,479,415, 6,465,379, 5,695,486, 6,533,898, 6,562,742, 6,562,743, 6,559,081, 6,495,734, 6,649,809, 6,420,626, 6,726,461, 6,035,608, 6,403,857, 6,479,415, 6,562,742, 6,562,743, 6,559,081, 6,495,734, 6,420,626 and in U.S. Patent applications with serial numbers and filing dates, 09/719,338 filed January 17, 2001; 09/774,248 filed January 30, 2001; 09/854,179 filed May 11, 2001, and 60/569,980 filed May 10, 2004, all of which are hereby incorporated by reference in their entirety.

With regard to the enzyme inhibitors used to treat fibers to produce the treated fibers of this invention, the enzyme inhibitor must be present on the fiber in an amount which is sufficient for the material to be effective, in other words, it must be present in an effective amount. To be effective, the enzyme inhibitor must be available to engage in enzyme inhibitory action. For example, in the case of metal ions which are used as enzyme inhibitors, it is not merely enough for the metal ion in the form of a salt to be coated on the fiber. When a personal care article incorporating the fiber is insulted with a liquid containing material, it is important for the metal ions to be able to be transported to and contact the enzyme so that it may be inactivated. Generally, but not always, this means that the metal ions coated on the fiber must be in a form that is soluble when contacted by aqueous materials or which becomes soluble under conditions of use. Alternatively, transport of inhibitor through a mechanism dependent on solubility may not be that important, if the inhibitor is on a support such as a fiber or functional particle and the enzyme is inhibited through a heterogeneous mechanism.

### Functional Particles

In another embodiment of this invention, superabsorbent polymer particles (SAP) may be used in combination with the treated fibers of this invention in various personal care articles, or the SAP particles or fibers, or other functional particles, can be treated with one or more enzyme inhibitors themselves and may then be used either by themselves or in combination with treated fibers in personal care articles. The treated SAP or other functional particles of this invention are produced by contacting the SAP or other functional particles with an enzyme inhibitor in an amount of from about 0.0001 % to about 50% by weight, typically about 0.001% to about 25%, more typically about 0.01% to about 10%, and most typically about 0.1 % to about 5%, and in various applications, depending on the enzyme inhibitor used, in ranges comparable to those suggested for the treated fibers of this invention, such as, for various metal ions, it is desirable that the metal ion be present and available in an amount from about 1 ppm (parts per million by weight) to about 10,000 ppm, more desirably from about 10 ppm to about 5,000 ppm and still more desirably, from about 100 ppm to about 1,000 ppm. For other enzyme inhibitors, for example, phosphoric triamide inhibitors, it is desirable that the compound be present and active in an amount of from abut 1 ppm to about 2,500 ppm, more desirably, in an amount of from about 10 ppm to about 1000 ppm, still more desirably, in an amount of from about 10 ppm to about 500 ppm.

Functional particles for use in the absorbent cores of the invention include particles, flakes, powders, granules or the like which usually serve as absorbents, zeolites or calcium carbonates. The particles may include any functional powder or other particle having a particle diameter up to 3,000µ (microns). In preferred embodiments, the particles are SAP.

U.S. Patent Nos. 5,147,343; 5,378,528; 5,795,439; 5,807,916; and 5,849,211, hereby incorporated herein by reference in their entirety, which describe various superabsorbent polymers and methods of manufacture, are hereby incorporated by reference. Examples of the types of SAP particles which may be used in this invention, include superabsorbent polymers in their particulate form such as irregular granules, spherical particles, staple fibers and other elongated particles. The term "superabsorbent polymer" or "SAP" refers to a normally water-soluble polymer, which has been cross-linked. There are known methods of making water-soluble polymers such as carboxylic polyelectrolytes to create hydrogel-forming materials, now commonly referred to as superabsorbents or SAPs, and it is well known to use such materials to enhance the absorbency of disposable absorbent articles. There are also known methods of crosslinking carboxylated polyelectrolytes to obtain superabsorbent polymers. SAP particles useful in the practice of this invention are commercially available from a number of manufacturers, including Dow Chemical (Midland, Michigan), Stockhausen (Greensboro, North Carolina), and Chemdal (Arlington Heights, Illinois). One conventional granular superabsorbent polymer is based on poly(acrylic acid) which has been crosslinked during polymerization with any of a number of multi-functional co-monomer crosslinking agents, as is well known in the art. Examples of multifunctional crosslinking agents are set forth in U.S. Patent Nos. 2,929,154; 3,224,986; 3,332,909; and 4,076,673, hereby incorporated herein by reference in their entirety. Other water-soluble polyelectrolyte polymers are known to be useful for the preparation of superabsorbents by crosslinking, these polymers include carboxymethyl starch, carboxymethyl cellulose, chitosan salts, gelatin salts, etc. They are not, however, commonly used on a commercial scale to enhance absorbency of disposable absorbent articles, primarily due to lower absorbent efficiency or higher cost.

Superabsorbent polymers are well known and are commercially available. Superabsorbent particulate polymers are also described in detail in U.S. Patent No. 4,102,340 and Re 32,649, hereby incorporated herein by reference in their entirety.

Suitable SAPs yield high gel volumes or high gel strength as measured by the shear modulus of the hydrogel. Such preferred SAPs contain relatively low levels of polymeric materials that can be extracted by contact with synthetic urine (so-called "extractables"). SAPs are well known and are commercially available from several sources. One example is a starch graft polyacrylate hydrogel marketed under the name IM1000 (Hoechst-Celanese; Portsmouth, VA). Other commercially available superabsorbers are marketed under the trademark SANWET (Sanyo Kasei Kogyo; Kabushiki, Japan), SUMIKA GEL (Sumitomo Kagaku Kabushiki; Haishi, Japan), FAVOR (Stockhausen; Garyville, LA) and the ASAP series (Chemdal; Aberdeen, MS). Most preferred for use with the present invention are polyacrylate-based SAPs. As used in the present invention, SAP particles of any size or shape suitable for use in an absorbent core may be employed.

Another aspect of this invention relates to a method of enhancing the yield and/or growth of plants by distributing the composition of this invention in the "plant growth medium" in which the plants are being grown within reach of the root system of the plants (hereinafter referred to as "root zone"). As herein, the term "plant growth medium" refers to various natural and artificial medium which support plant growth, including but not limited to soil, potting mixtures of organic and inorganic matter and artificial medium such as polyurethane foam.

### Enzyme Inhibiting Spray

This invention also provide an enzyme inhibiting spray for house and garden use. The spray consists of a mechanical sprayer or an aerosol sprayer which, when loaded with spray solution, has a mass of 2.5 kg or less, preferable, of 1 kg or less and more preferably of 0.5 kg or less. The mechanical sprayer or aerosol sprayer are of any suitable type know in the art and commonly available. The spray solution is an aqueous solution of an available enzyme inhibitor. The sprayer may be used in the home or garden to spray pet litter boxes or devices used by the pet, or areas of the yard or garden when a pet is accustomed to eliminating an odor causing material. Spraying these areas and materials either before or after used by the pet with the spray solution has the effect of reducing the generation of odor from the deposit relative to what would be generated without the spray. The concentration of the enzyme inhibitor in the spray solution is from about 0.0001 weight percent to about 10 weight percent of an available enzyme inhibitor.
Within the scope of this aspect of the invention are containers of spray solution containing about 40 liters or less of spray solution, more typically, about 10 liters or less, often of about 4 liters or less.

### EXAMPLES

The present invention will be better understood by reference to the following Examples, which are provided as exemplary of the invention, and not by way of limitation.

### MATERIALS

1. VIZORB® 3905 available from Buckeye Technologies Inc. of Memphis, Tennessee. VIZORB® 3905 is a type of multibonded airlaid nonwoven product having a basis weight of 250 gsm and containing southern softwood kraft cellulosic fibers and superabsorbent polymer particles in its composition.
   VIZORB® 3910 available from Buckeye Technologies Inc. of Memphis, Tennessee. VIZORB® 3910 is a type of multibonded airlaid nonwoven product having a basis weight of 175 gsm and containing southern softwood kraft cellulosic fibers and superabsorbent polymer particles in its composition.
2. FOLEY FLUFFS® available from Buckeye Technologies Inc. of Memphis, Tennessee. FOLEY FLUFFS® is southern softwood bleached kraft cellulose pulp.
3. N-(n-butyl) thiophosphoric triamide (NBPT) obtained from Agrotain International LLC of Corydon, Kentucky. NBPT is a commercially available urease inhibitor, which is used in agriculture.
4. Chemical grade zinc chloride.
5. Commercial solution of aluminum sulfate in water. The concentration of aluminum sulfate is about 48%.
6. Solution of urea and sodium chloride in demineralized water. The concentration of urea is adjusted to 2 % by weight of the solution and the concentration of sodium chloride is 0.9% by weight of the solution.
7. Urine collected from healthy adult persons.
8. U1875 - a solution of jack bean urease in glycerol purchased from Sigma-Aldrich. The concentration of urease in this solution is 50% by weight. The solution has an activity of 500-800 units/ml. One unit is defined as an amount of urease, which will liberate 1.0 µmole of NH₃ from urea per min at pH 7.0 at 25 °C. It is equivalent to 1.0 I.U. or 0.054 Sumner unit (1.0 mg ammonia nitrogen in 5 minutes at pH 7.0 at 20°C)

### EXAMPLE 1: Effect of VIZORB® 3905 treated with zinc and aluminum salts on ammonia generation from the solution of urea

VIZORB® 3905 material was cut to rectangular sheets, approximately 10 cm by 20 cm so that each sheet weighed 5 grams. In order to obtain desired levels of aluminum and of zinc on the sheets appropriate amounts of either concentrated aluminum sulfate solution at 48% concentration by weight was diluted in demineralized water or solid zinc chloride was dissolved in demineralized water to make up the solutions for spraying. These amounts are listed in Table 1. Each sheet was then sprayed on both sides with a total of about 15 mL solution of the pre-weighed chemical according to the amount specified in Table 1 and left to dry in ambient conditions to not more than 10% moisture content.

**Table 1**

| Amount of aluminum sulfate solution at 48%, g per 10 grams of VIZORB® 3905 | Aluminum add-on, ppm | Amount of solid zinc chloride, g per 10 grams of VIZORB® 3905 | Zinc add-on, ppm |
|---|---|---|---|
| 0.92 | 7000 | 0.11 | 5000 |
| 1.31 | 10000 | 0.13 | 6000 |
| 1.84 | 14000 | 0.21 | 10000 |

Some sheets of the VIZORB® 3905 material were not sprayed with any solutions and served as control substrates.

10 grams of each type of the control and the treated VIZORB® 3905 sheets were placed in hermetic plexi-glass containers as shown in Figure 1. The dimensions of each container were the following: length 20 cm, width 10 cm and height 12 cm. Each container had two outlets in the lid (1), one (2) having a diameter of about 1 cm and the other (3) having a diameter of about 2.5 cm, with rubber stoppers. The bigger opening served to insult the sheets in the container with the test liquid. The smaller opening was big enough just to insert a Draeger tube attached to the gas detector pump for measuring the concentration of ammonia in the container during the test. The highest concentration that can be measured with the aid of this equipment is 700 ppm. The gas detector pump and the tubes were purchased from Draeger Safety Inc., Pittsburgh, Pennsylvania.

The containers with the VIZORB® 3905 sheets (4) were placed in a water bath maintained at 37°C. The solution of urea and sodium chloride in demineralized water was divided into aliquots of 100 mL. The U 1875 solution of urease in an amount of 0.2 mL was then added to each 100 mL aliquot and the synthetic urine was used to insult the test sheets placed in the containers. After insulting the sheets, the opening in the container was sealed with the stopper and the timer was turned on. The concentration of ammonia was measured at various time intervals during the experiment. In order to measure the concentration of ammonia in the air in the container above the sample, the smaller outlet was opened and an Ammonia 5/a Draeger tube was inserted through it to the container to half of the length of the tube. The measurement was performed according to the instructions supplied by the producer of the Draeger tubes. After the measurement the small outlet in the container was sealed again with the stopper for the continuation of the experiment. The results of the tests are shown in Table 2. The data in Table 2 are concentrations of ammonia in ppm in the containers.

The data shown in Table 2 indicate that both Al and Zn have an inhibitory effect on the emission of ammonia. In this experiment the efficiency of Zn was higher that that of Al.

**Table 2 Ammonia Generated in PPM**

| Time, hours | Control | Amount of Al, ppm, on Vizorb material | | | Amount of Zn, ppm, on Vizorb material | | |
|---|---|---|---|---|---|---|---|
| | | 7000 | 10000 | 14000 | 5000 | 6000 | 10000 |
| 0.5 | 20 | 5 | 5 | 0 | 5 | 5 | 5 |
| 1.0 | 100 | 20 | 10 | 5 | 20 | 20 | 5 |
| 1.5 | 250 | 60 | 30 | 10 | 40 | 50 | 40 |
| 3 | 600 | 300 | 125 | 70 | 100 | 100 | 80 |
| 7 | 700 | 700 | 500 | 500 | 230 | 200 | 150 |
| 23 | | | 700 | 700 | 350 | 300 | 300 |

### EXAMPLE 2: Effect of VIZORB® 3905 treated with NBPT on ammonia generation from the solution of urea

A 0.015 weight percent solution of NBPT in water was prepared and sprayed on 10 g of VIZORB® 3905, which was two handsheets having dimensions of 20 cm by 10 cm, in an amount of 1 mL of solution per gram of VIZORB® 3905 to obtain an add-on level of NBPT at 150 ppm. This procedure was followed by drying the sprayed sheets at ambient temperature. Some sheets were not treated and they served as control samples for the experiments. Both treated and untreated VIZORB® 3905 sheets were placed in the test containers as described in Example 1. The test solution of urea and sodium chloride in demineralized were mixed each with 0.8 mL of the U 1875 urease solution per 200 mL of the test solution. Aliquots of 200mL of the resultant solutions were used to insult the VIZORB® 3905 sheets in each test container, which had been previously equilibrated to a temperature of 37°C. The small outlet in the containers was opened quickly at defined time intervals to check for the smell of ammonia. The results are shown in Table 3.

**Table 3**

| Sample | 40 min | 23 hrs | 150 hrs |
|---|---|---|---|
| Control, untreated VIZORB® 3905 | ++ | ++ | ++ |
| VIZORB® 3905 treated with NBPT at 150 ppm | - | + + | ++ ++ |

| | | | |
|---|---|---|---|
| - no smell of ammonia | | | |
| + slight smell of ammonia | | | |
| ++ strong smell of ammonia | | | |

The data in the Table 3 suggest that the VIZORB® composite treated with NBPT effectively reduced ammonia odor.

### EXAMPLE 3: Effect of VIZORB® 3905 treated with NBPT, zinc chloride and aluminum sulfate on ammonia generation from urine

VIZORB® 3905 handsheets were treated to obtain various levels of the chemicals as described in Examples 1 and 2 to get add-on levels of theses compounds on VIZORB® 3905 as indicated in Table 4. Untreated sheets of VIZORB® 3905 served as control samples for the experiments. Both treated and untreated VIZORB® 3905 sheets were placed in the test containers as described in Examples 1 and 2. Urine was collected from healthy adult persons and mixed with 0.1 mL of the U 1875 urease solution per 100 mL of urine. Aliquots of 100 mL of the resultant liquid were then used to insult the VIZORB® 3905 sheets in each test container, which had been equilibrated previously to a temperature of 37°C. The small outlet in the containers was opened quickly at defined time intervals to check the concentration of ammonia with the Draeger tubes. The results are shown in Table 4.

**Table 4**

| Time, hrs | Control | NBPT at 200 ppm | NBPT at 800 ppm | Zn at 7000 ppm | A1 at 7000 ppm | Temp., °C |
|---|---|---|---|---|---|---|
| 0.5 | 10 | 0 | 0 | 0 | 0 | 37 |
| 1 | 40 | 0 | 0 | 0 | 0 | 37 |
| 2 | 250 | 0 | 0 | 15 | 50 | 37 |
| 3 | 600 | 0 | 0 | 90 | 275 | 37 |
| 4 | | 0 | 0 | 200 | 550 | 37 |
| 5 | | 0 | 0 | 325 | >700 | 37 |
| 6 | | 0 | 0 | 350 | | 30 |
| 9 | | 0 | 0 | 225 | | 25 |
| 24 | | 0 | 0 | 600 | | 37 |

The data shown in Table 4 suggest that all the treated samples reduced the rate of ammonia emission. The samples treated with NBPT were the most effective whereas the sample treated with aluminum sulfate was the least effective.

After two hours, when compared to the control, the sample with Zn at 7000 ppm showed a reduction of ammonia concentration to 4%, the A1 at 7000 ppm showed a reduction of ammonia concentration to 20%, while the samples with NBPT showed a reduction to 0%. All of these samples of treated fibers of this invention meet the criterion of having available enzyme inhibitor, although the sample with Zn is more effective as a urease inhibitor than the sample with Al, both at 7000 ppm, while the samples with NBPT are available and more effective than either metal ion, even at only 200 ppm.

### EXAMPLE 4: Effect of VIZORB® 3905 and FOLEY FLUFFS® treated with NBPT and zinc chloride on ammonia generation from urine.

VIZORB® 3905 handsheets were treated to obtain various levels of the chemicals as described in Examples 1, 2 and 3 to get add-on levels of theses compounds on VIZORB® 3905 as indicated in Table 5. FOLEY FLUFFS® pulp sheet was disintegrated in a laboratory fiberizer and used in the fluff form to make 250 gsm handsheets in a laboratory dry handsheet former. Thus prepared sheets were densified and cut smaller sheets each having a rectangular size of 20 cm by 10 cm. Some of these sheets were treated with the chemicals in a way similar to that used in the case of the VIZORB® 3905 sheets in order to obtain the treated samples with either NBPT or with zinc chloride at add-on levels indicated in Table 5. The untreated sheets of VIZORB® 3905 as well as the untreated sheets of FOLEY FLUFFS® served as control samples for the experiments. Both treated and untreated sheets of VIZORB® 3905 and of FOLEY FLUFFS® were placed in the test containers as described in Examples 1, 2 and 3. Urine was collected from healthy adult persons and mixed with 0.1 mL of the U 1875 urease solution per 100 mL of urine. Aliquots of 80 mL of the resultant liquid were then used to insult the VIZORB® 3905 sheets whereas aliquots of 50 mL were used to insult the FOLEY FLUFFS® sheets in the test containers, which had been equilibrated previously to a temperature of 37°C. The small outlet in the containers was opened quickly at defined time intervals to check the concentration of ammonia with the Draeger tubes. The results are shown in Table 5.

The data shown in Table 5 suggest that all the treated samples reduced the rate of ammonia emission.

**Table 5**

| Time, hrs | Untreated VIZORB® 3905 | Untreated FOLEY FLUFFS® | VIZORB® 3905 treated with NBPT at 100 ppm | VIZORB® 3905 treated with NBPT at 200 ppm | FOLEY FLUFFS® treated with NBPT at 200 ppm |
|---|---|---|---|---|---|
| 0.5 | 10 | 30 | 0 | 0 | 5 |
| 1 | 50 | 170 | 0 | 0 | 10 |
| 2 | 170 | 400 | 0 | 0 | 20 |
| 3 | 400 | 700 | 0 | 0 | 20 |
| 4 | 500 | | 0 | 0 | 40 |
| 5 | 700 | | 0 | 0 | 50 |
| 9 | | | 0 | 0 | 150 |
| 24 | | | 10 | 0 | 600 |

### EXAMPLE 5: Treated FOLEY FLUFFS® for Odor Control

FOLEY FLUFFS® is comminuted into individualized fluff pulp fibers, samples of which are then treated with NBPT solution, zinc chloride solution or aluminum sulfate solution, respectively. Each of the treated FOLEY FLUFFS® is then used to prepare handsheet material similar to VIZORB® , but without superabsorbent material. These handsheets are then tested as were the sheets in Examples 1-4, and are found to be similarly effective in controlling the generation of ammonia from urine.

### EXAMPLE 6: Effect of FOLEY FLUFFS® and FAVOR® SXM1180 composites treated with NBPT on ammonia generation from urine

A FOLEY FLUFFS® pulp handsheet at a basis weight of about 700 gsm and in an amount of 34.5 g was sprayed with 30 mL aqueous solution containing 0.0138 g NBPT in demineralized water. The sheet was air-dried to a moisture content not higher than 10% by weight. It was then disintegrated in a laboratory fiberizer to produce the fluff containing 400 ppm NBPT. A portion of this fluff was blended with untreated FOLEY FLUFFS® to produce the fluff containing 200 ppm NBPT. Various composite handsheets were made using the prepared, treated fluff samples and FAVOR® SXM1180 at various ratios of the fluff to FAVOR® SXM1180. The handsheets were formed on a laboratory handsheet dry former, each at a basis weight of 250 gsm and densified to 0.2g/cm³. The compositions of the handsheets 1-5 are shown in Table 6.

**Table 6**

| | Percent content of various components in handsheets 1-5 | | | | |
|---|---|---|---|---|---|
| Component | 1 | 2 | 3 | 4 | 5 |
| Untreated FOLEY FLUFFS® | | | | | 50 |
| FOLEY FLUFFS® with 200 ppm NBPT | 100 | 85 | 70 | 50 | |
| FAVOR® SXM1180 | | 15 | 30 | 50 | 50 |

The handsheets 1-5 were cut to smaller test sheets each having a rectangular size of 20 cm by 10 cm. They were then placed in the test containers as described in Examples 2-5. Urine was collected from healthy adult persons and mixed with 0.1 mL of the U1875 urease solution per 100 mL of urine. Aliquots of 50 mL of the resultant liquid were then used to insult the test sheets in the containers, which had been equilibrated previously to a temperature of 37°C. The small outlet in the containers was opened quickly at defined time intervals to check the concentration of ammonia with the Draeger tubes. The results are shown in Table 7. The data in Table 7 are concentrations of ammonia in ppm in the test containers.

The data shown in Table 7 suggest that all the test sheets containing NBPT effectively inhibited the emission of ammonia, while the sheet which did not contain NBPT did not inhibit emission of ammonia.

**Table 7**

| Time, hrs | 1 | 2 | 3 | 4 | 5 |
|---|---|---|---|---|---|
| 1 | 3 | 0 | 0 | 0 | 5 |
| 2 | 4 | 0 | 0 | 0 | 20 |
| 3 | 4 | 0 | 0 | 0 | 50 |
| 4 | 5 | 0 | 0 | 0 | 60 |
| 5 | 5 | 0 | 0 | 0 | 175 |
| 20 | 100 | 50 | 70 | 70 | >700 |

Patents, patent applications, publications, product descriptions, and protocols are cited throughout this application, the disclosures of which are incorporated herein by reference in their entireties for all purposes.

### EXAMPLE 7: Effect of VIZORB® 3910 treated with aluminum and zinc compounds on ammonia generation from urine

VIZORB® 3910 handsheets were treated with various aluminum and zinc compounds to prepare samples 1-5 for the ammonia emission test.
Sample 1: Two VIZORB® 3910 rectangular sheets having dimensions of about 10 cm by 20 cm and weighing 10 grams were used as a control substrate in the experiment.
Sample 2: Two VIZORB® 3910 rectangular sheets having dimensions of about 10 cm by 20 cm and weighing 10 grams were sprayed with an aqueous solution of aluminum sulfate at a concentration of 2.5% by weight in an amount sufficient to introduce 10,000 ppm of aluminum on the VIZORB® 3910 material. Subsequent to that the sheets were sprayed with an aqueous solution of sodium hydroxide at a concentration of about 1.5% by weight in an amount sufficient to introduce 0.24 g sodium hydroxide on 10 grams of the VIZORB® 3910 sheets. The treated sheets were dried in an oven at 105°C until the moisture content in the sheets was not higher than 10% by weight.
Sample 3: Two VIZORB® 3910 rectangular sheets having dimensions of about 10 cm by 20 cm and weighing 10 grams were prepared in the same way as the sheets of Sample 2. Then these sheets were additionally sprayed with an aqueous solution of citric acid at a concentration of 1.5% by weight in an amount sufficient to introduce 0.24 g citric acid on 10 grams of the VIZORB® 3910 material. The treated sheets were dried in an oven at 105°C until the moisture content in the sheets was not higher than 10% by weight.
Sample 4: Two VIZORB® 3910 rectangular sheets having dimensions of about 10 cm by 20 cm and weighing 10 grams were sprayed with an aqueous solution of zinc sulfate heptahydrate at a concentration of about 2.5% by weight in an amount sufficient to introduce 10,000 ppm of zinc on the VIZORB® 3910 material. Subsequent to that the sheets were sprayed with an aqueous solution of sodium hydroxide at a concentration of about 1.0% by weight in an amount sufficient to introduce 0.12 g sodium hydroxide on 10 grams of the VIZORB® 3910 sheets. The treated sheets were dried in an oven at 105°C until the moisture content in the sheets was not higher than 10% by weight.
Sample 5 Two VIZORB® 3910 rectangular sheets having dimensions of about 10 cm by 20 cm and weighing 10 grams were sprayed with an aqueous solution of zinc sulfate heptahydrate at a concentration of about 2,5% by weight in an amount sufficient to introduce 10,000 ppm of zinc on the VIZORB® 3910 material. Thus treated sheets were dried in an oven at 105°C until the moisture content in the sheets was not higher than 10% by weight.

The Samples 1-5 were placed in the test containers as described in the previous Examples. Urine was collected from healthy adult persons and mixed with 0.1 mL of the U1875 urease solution per 100 mL of urine. Aliquots of 80 mL of the resultant liquid were then used to insult the Samples 1-5 in each test container, which had been equilibrated previously to a temperature of 37°C. The small outlet in the containers was opened quickly at defined time intervals to check the concentration of ammonia with the Draeger tubes. The results are shown in Table 8. The data in Table 8 are concentrations of ammonia in ppm in the containers.

**Table 8 Ammonia Concentration in ppm**

| Time, hrs | 1 | 2 | 3 | 4 | 5 |
|---|---|---|---|---|---|
| 1 | 100 | 100 | 70 | 80 | 20 |
| 2 | 300 | 300 | 200 | 90 | 40 |
| 3 | 500 | 500 | 400 | 125 | 60 |
| 4 | >700 | 600 | 500 | 150 | 60 |
| 6 | | >700 | 700 | 200 | 60 |
| 9 | | | | 250 | 70 |
| 24 | | | | 300 | 140 |

The data in Table 8 suggest that increasing the solubility of the metal-containing compound leads to an increase of its inhibitory effect on the ammonia emission. Without being bound to theory it is hypothesized that the differences in the inhibitory effects observed in Samples 2-5 were related to the availability of the tested metal cations to the enzyme urease which decomposes urea into ammonia. Sample 2 contained insoluble aluminum hydroxide as a result of the neutralization of aluminum sulfate with sodium hydroxide. Sample 3 contained aluminum hydroxide precipitated on the material in the same manner as in Sample 2. However, Sample 3 contained also citric acid, which reduces the pH of the sample and may potentially convert part of the aluminum hydroxide into a soluble salt of aluminum. Apparently, such conversion occurred in the applied experimental conditions to a small extent and therefore the increase in the availability of aluminum cations to the enzyme was still very limited, as illustrated by the data in Table 8. Since the reduction in the ammonia concentration in comparison to the control at two hours after the insult was only to 67 percent, it cannot be said that the substrate treated in this way has an available enzyme inhibitor.

Sample 4 contained zinc hydroxide whose solubility is much lower than the solubility of zinc sulfate deposited on Sample 5. Therefore, the availability of zinc cations to the enzyme in Sample 5 was much higher than in Sample 4. As a result Sample 5 was much more efficient in inhibiting the emission of ammonia than Sample 4. Sample 4, with a two hour reduction of ammonia concentration relative to the control to 30 percent, has an available enzyme inhibitor with an inhibitory effectiveness of 70 percent, while Sample 5, with a reduction to 13 percent has an available enzyme inhibitor, and it can be said that it is more effective than Sample 4, since it has an inhibitory effectiveness of 87 percent.

### EXAMPLE 8: Effect of FOLEY FLUFFS® treated with ammonium thiosulfate (ATS) on ammonia generation from urine

An aqueous solution of ATS having a concentration of 55% was treated with a small amount of zinc chloride aqueous solution having a concentration of 62.5%. The zinc chloride solution was added to the ATS solution with constant agitation in an amount sufficient to bring the pH of the mixture to 6.5. By doing this the ammonia odor of the ATS solution was reduced to a level hardly detectable by smell. The obtained solution was diluted with various amounts of water to get three separate solutions having various concentrations of ATS. These concentrations were: (1) about 11 %, (2) about 20% and (3) about 28%. The obtained solutions were used then in various amounts to treat respectively three sheets of FOLEY FLUFFS® having a basis weight of about 700 gsm and weighing about 51 g. The amount of solution (1) used for spraying the first sheet was 4.61 g, the amount of solution (2) used for spraying the second sheet was 5.18 g, and the amount of solution (3) used for spraying the third sheet was 5.67 g. The sheets were air-dried to a moisture content not higher than 10% by weight. Each of the sheets was then disintegrated in a laboratory fiberizer to produce the fluff containing various amounts of ATS. The first fluffed sample contained about 1% ATS, the second fluffed sample contained about 2% ATS and the third fluffed sample contained about 3% ATS. Various composite handsheets were subsequently made using FAVOR® SXM1180 and the prepared, fluffed samples containing ATS as well as a control fluffed sample of FOLEY FLUFFS®. The weight ratio of the fluffed samples to FAVOR® SXM1180 was 80 : 20. The handsheets were formed on a laboratory handsheet dry former, each at a basis weight of 250 gsm and densified to 0.2g/cm³.

All the handsheets were cut to smaller test sheets each having a rectangular size of 20 cm by 10 cm. They were then placed in the test containers as described in Examples 2-5. Urine was collected from healthy adult persons and mixed with 0.1 mL of the U 1875 urease solution per 100 mL of urine. Aliquots of 80 mL of the resultant liquid were then used to insult the test sheets in the containers, which had been equilibrated previously to a temperature of 37°C. The small outlet in the containers was opened quickly at defined time intervals to check the concentration of ammonia with the Draeger tubes. The results are shown in Table 9. The data in Table 9 are concentrations of ammonia in ppm in the test containers.

The data shown in Table 9 suggest that all the test sheets containing ATS effectively reduced the emission of ammonia, when compared to the amount of ammonia emitted from the sheet which did not contain ATS.

**Table 9**

| Time, hrs | Ammonia emitted (in ppm) from composite handsheets containing cellulose with various ATS add-ons in weight percent and ppm, based on weight of pulp | | | Ammonia emitted (in ppm) from control composite |
|---|---|---|---|---|
| | 1% 10,000 ppm | 2% 20,000 ppm | 3% 30,000 ppm | |
| 1 | 0 | 0 | 0 | 10 |
| 2 | 0 | 0 | 3 | 30 |
| 3 | 5 | 3 | 5 | 70 |
| 4 | 5 | 5 | 10 | 120 |
| 5 | 5 | 5 | 15 | 250 |
| 6 | 10 | 15 | 15 | 400 |
| 10 | 15 | 25 | 20 | >700 |
| 21 | 20 | 40 | 40 | |
| 22 | 20 | 50 | 40 | |

### EXAMPLE 9: Effect of FAVOR® SXM1180 treated with NBPT on ammonia generation from urine

NBPT was dissolved in various amounts in methanol and three solutions were obtained of which the concentrations were: 0.1g NBPT in 100 mL methanol, 0.2 g NBPT in 100 mL methanol and 0.2 g NBPT in 100 mL methanol. These solutions were used for treating 20-gram samples of FAVOR® SXM1180. The solutions were applied in predetermined amounts to obtain three samples of FAVOR® SXM1180 having various contents of NBPT, that is 100 ppm, 200 ppm and 400 ppm, respectively. The treated SAP samples were air-dried overnight in ambient conditions. These SAP samples and a sample of untreated FAVOR® SXM1180 were then used for making composite handsheets with fluffed FOLEY FLUFFS®. The weight ratio of the SAP samples to fluff was 70 : 30. The handsheets were formed on a laboratory handsheet dry former, each at a basis weight of 250 gsm and densified to 0.2g/cm³.

All the handsheets were cut to smaller test sheets each having a rectangular size of 20 cm by 10 cm. They were then placed in the test containers as described in Examples 2-5. Urine was collected from healthy adult persons and mixed with 0.1 mL of the U1875 urease solution per 100 mL of urine. Aliquots of 80 mL of the resultant liquid were then used to insult the test sheets in the containers, which had been equilibrated previously to a temperature of 37°C. The small outlet in the containers was opened quickly at defined time intervals to check the concentration of ammonia with the Draeger tubes. The results are shown in Table 10. The data in Table 10 are concentrations of ammonia in ppm in the test containers.

The data shown in Table 10 suggest that all the test sheets containing SAP treated with NBPT effectively reduced the emission of ammonia, when compared to the amount of ammonia emitted from the sheet which contained untreated SAP. It can also be seen that the increase in the content of NBPT in the SAP results in a decrease of the concentration of ammonia emitted from the tested samples.

**Table 10**

| Time, hrs | Ammonia emitted (in ppm) from composite handsheets containing FAVOR® SXM1180 with NBPT at various contents | | | |
|---|---|---|---|---|
| | 0 ppm | 100 ppm | 200 ppm | 400 ppm |
| 1 | 50 | 10 | 10 | 10 |
| 2 | 130 | 30 | 20 | 5 |
| 3 | 300 | 50 | 20 | 5 |
| 4 | 500 | 75 | 20 | 5 |
| 5 | 600 | 100 | 20 | 5 |
| 6 | 700 | 100 | 20 | 5 |
| 10 | | 200 | 10 | 5 |
| 22 | | 700 | 10 | 0 |

### EXAMPLE 10: Effect of FOLEY FLUFFS® treated with zinc sulfate on ammonia generation from urine

Cellulose pulp sheet containing about 0.8% zinc applied in the form of zinc sulfate was made on a commercial paper machine and then a sample of it was fluffed in a lab disintegrator. The obtained fluff containing Zn as well as a control fluffed sample of FOLEY FLUFFS® were used for making composite handsheets with FAVOR® SXM1180. The weight ratio of the fluffed samples to FAVOR® SXM1180 was 80 : 20. The handsheets were formed on a laboratory handsheet dry former, each at a basis weight of 250 gsm and densified to 0.2g/cm³.

All the handsheets were cut to smaller test sheets each having a rectangular size of 20 cm by 10 cm. They were then placed in the test containers as described in Examples 2-5. Urine was collected from healthy adult persons and mixed with 0.1 mL of the U1875 urease solution per 100 mL of urine. Aliquots of 80 mL of the resultant liquid were then used to insult the test sheets in the containers, which had been equilibrated previously to a temperature of 37°C. The small outlet in the containers was opened quickly at defined time intervals to check the concentration of ammonia with the Draeger tubes. The results are shown in Table 11. The data in Table 11 are concentrations of ammonia in ppm in the test containers.

The data shown in Table 11 suggest that the test sheet containing zinc effectively reduced the emission of ammonia, when compared to the amount of ammonia emitted from the sheet which did not contain zinc.

The same cellulose containing zinc was used to make adult incontinence (AI) diapers, which were then tested both in the lab for ammonia emission and in nursing homes for odor reduction. Both the lab results, which are shown in Table 12, and use test data indicated that the diapers containing the cellulose with zinc produced significantly less ammonia and less odor, respectively, than the diaper containing regular cellulose fluff.

## Claims

1. A treated fiber comprising fiber and based on the weight of the treated fiber from about 0.0001 weight percent to about 10 weight percent of an available enzyme inhibitor.

2. The treated fiber of claim 1 in individualized form.

3. The treated fiber of claim 1 in the form of a comminution sheet.

4. The treated fiber one of the previous claims, wherein the enzyme inhibitor is an inhibitor of proteolytic enzymes.

5. The treated fiber of claim 4, wherein the inhibitor of proteolytic enzymes inhibits trypsin, chymotrypsin, aminopeptidase, elastase, lipases, bile salts, amylases, ureases, or a combination thereof.

6. The treated fiber of one of the previous claims, wherein the enzyme inhibitor is a chelating agent.

7. The treated fiber of one of the previous claims, wherein the enzyme inhibitor is a protease inhibitor, a lipase inhibitor, a bile salt inhibitor, an amylase inhibitor, a glucosidase inhibitor, or a combination thereof.

8. The treated fiber of one of the previous claims, wherein the enzyme inhibitor is a urease inhibitor.

9. The treated fiber of one of the previous claims, wherein the enzyme inhibitor is a metal salt.

10. The treated fiber of one of the previous claims, wherein the enzyme inhibitor is a transition metal ion salt.

11. The treated fiber of one of the previous claims, wherein the enzyme inhibitor is a salt comprising zinc or aluminum ions.

12. The treated fiber of one of the previous claims, wherein the enzyme inhibitor is a salt comprising zinc ions.

13. The treated fiber of one of the previous claims, wherein the metal salt has a metal ion content of from about 0.0005 weight percent to about 5 weight percent based on the weight of the treated fiber.

14. The treated fiber of one of the previous claims, wherein the metal salt has a metal ion content of from about 0.0005 weight percent to about 3 weight percent based on the weight of the treated fiber.

15. The treated fiber of one of the previous claims, wherein the metal salt has a metal ion content of from about 0.0005 weight percent to about 2 weight percent based on the weight of the treated fiber.

16. The treated fiber of one of the previous claims, wherein the metal salt has a metal ion content of from about 0.001 weight percent to about 2 weight percent based on the weight of the treated fiber.

17. The treated fiber of one of the previous claims, wherein the metal salt has a metal ion content of from about 0.01 weight percent to about 2 weight percent based on the weight of the treated fiber.

18. The treated fiber of one of claims 9-12, wherein the metal salt is present in an amount from about 10 ppm to about 10,000 ppm.

19. The treated fiber of one of the previous claims, wherein the enzyme inhibitor is zinc chloride, zinc sulfate or a mixture thereof.

20. The treated fiber of one of the previous claims, wherein the enzyme inhibitor is ammonium thiosulfate.

21. The treated fiber of one of the previous claims, wherein the enzyme inhibitor is a phosphoric triamide compound.

22. The treated fiber of claim 21, wherein the phosphoric triamide compound is present in an amount of from about 1 ppm to about 2,500 ppm.

23. The treated fiber of one of the previous claims, wherein the inhibitory effectiveness of the treated fiber is about 75 percent or greater.

24. A process for the production of a treated fiber comprising contacting fibers with from about 0.0001 weight percent to about 10 weight percent of an enzyme inhibitor, based on the weight of the treated fiber, to produce a treated fiber having an available enzyme inhibitor.

25. The process of claim 24, wherein the fibers are in individualized form when contacted with the enzyme inhibitor.

26. The process of claim 24, wherein the fibers are in the form of a comminution sheet when contacted with the enzyme inhibitor.

27. The process of claim 24, wherein the fibers are in the form of a nonwoven material when contacted with the enzyme inhibitor.

28. A treated functional particle comprising a functional particle and based on the weight of the treated functional particle from about 0.0001 weight percent to about 10 weight percent of an available enzyme inhibitor.

29. The treated functional particle of claim 28, wherein the treated functional particle is a SAP particle.

30. A process for the production of a treated functional particle comprising contacting functional particles with from about 0.0001 weight percent to about 10 weight percent of an enzyme inhibitor based on the weight of the treated functional particle.

31. An absorbent structure comprising
(A) a treated fiber comprising fiber and based on the weight of the treated fiber from about 0.0001 weight percent to about 10 weight percent of an available enzyme inhibitor,
(B) a binder, and
(C) optionally, functional particles.

32. The absorbent structure of claim 31, wherein the structure is an airlaid nonwoven structure.

33. A sprayer comprising
(A) a mechanical or aerosol sprayer, and
(B) spray solution containing from about 0.0001 weight percent to about 10 weight percent of an available enzyme inhibitor.

34. The sprayer of claim 33, wherein the sprayer has a mass of 2.5 kg or less.
